# EUROPEAN PATENT APPLICATION

(11) **EP 1 504 722 A2**
(43) Date of publication of application: **09.02.2005**
(21) Application number: 04425531.3
(22) Date of filing: 15.07.2004
(51) Int. Cl.: A61B 10/00

(54) **Device for drawing biological and/or chemical samples**

(30) Priority: 08.08.2003 IT RN20030024
(71) Applicant: Genefast S.r.l., 41100 Modena (IT)
(72) Inventor: Turba, Maria Elena, 40100 Bologna (IT)
(74) Representative: Lanzoni, Luciano

(57) **Abstract**

A device for drawing biological and/or chemical samples, comprising a container (1) and a drawing head (2) that can be at least partially inserted into the container (1) and is movable at least between a position of readiness for sampling, external to the container (1) and a resting position internal to the container (1), further comprises means (3) for the guided manoeuvring o f the head (2), manoeuvring the head (2) in guided fashion relative to the container (1) between the position of readiness for sampling and the resting position.

## Description

The present invention relates to a device for drawing biological and/or chemical samples, in particular for use in the medical, biological, veterinary, forensic and industrial sectors.

The techniques used to draw and collect biological and/or chemical matrices generally entail use of a probe, usually constituted by swabs of brushes of adsorbent and/or abrasive material, aimed at collecting (generally by swiping and/or contacting on the involved part) of quantities of biological and/or chemical material that are useful and sufficient for subsequent laboratory tests.

Depending on the type of investigation and/or test to be subsequently performed on the drawn sample, the swab/brush can be preserved, after the drawing, in an isolated environment, occasionally enriched with transport and/or culture media or means.

Tools currently used normally for drawing biological and/or chemical material entail the use of rods of various lengths, provided, at one of their ends, with swabs/brushes. After collecting the biological or chemical material, the rods, with their swab/brush, are positioned inside test tubes or suitable containers, whose length generally corresponds to that of the rod in use.

There are also rods provided with swabs whose grip, positioned at the opposite end relative to the swab, serves as a plug for the test tube into which the rod (with its swab) is positioned after the drawing.

The rod equipped with the swab is usually supplied together with the test tube, already inserted therein. The user must, therefore, extract the rod from the test tube by means of the grip (thus opening the test tube), set down the empty test tube somewhere and proceed with swiping/drawing biological material. Once the drawing operation is complete, the user must take and hold the test tube with one hand, whilst with the other hand (s)he reinserts the rod, bearing the drawn biological sample on the swab, into the test tube. The test tube is lastly closed again by the grip of the rod, which serves as a plug.

Traditional collection swabs/brushes described above have some drawbacks.

In particular, the step of repositioning the probe (swab or brush) into a protecting structure (the test tube), which must necessarily be sealed to avoid contamination, is difficult. There is a concrete risk of making a mistake when inserting the rod which bears the swab into the test tube, and the risk increases when the rod is long. Moreover, it is not unlikely to risk touching the swab somewhere before the drawing operation, thereby contaminating it.

Therefore, there is a possibility that the swab may be contaminated both during its extraction and during its positioning in the test tube (particularly important when the rod of the swab is long). There is a concrete likelihood that the operator, or other surfaces or objects may be contaminated by the drawn material on the swab. There is a possibility that the collected material may be released, as a consequence of the contact of the swab with objects, surfaces and/or persons before its reinsertion into the test tube. Also during the step of repositioning the rod provided with the swab into the test tube, there is a concrete possibility of c ontaminating the outer w alls of the test tube w ith the material borne by the swab.

Given the correspondence between the dimensions of the test tube and those of the rod with the swab, which must be completely contained within the test tube, known system provide for the use of a large quantity of plastic material (for test tube and rod) and of an equally large quantity of any transport and/or culture media inserted in the test tube itself.

Moreover, during multiple sampling operations (simultaneous or timed closely to each other) there is a critical step for identifying the sample, which corresponds to the time between the drawing operation and the subsequent reallocation of the rod into the corresponding test tube. Although it is easy to identify the test tube with an appropriate code before executing the drawing operation, on the contrary a swab and its rod do not have surfaces suitable to affix any label bearing a code. The resulting risk of misidentifying the sample is particularly severe in the veterinary sector, where very numerous multiple samplings are daily fare and are conducted in the same location even by several persons at the same time, with the risk of mixing up swabs and test tubes.

The object of the present invention is to overcome the drawbacks described above, making available a device for drawing biological and/or chemical samples, which allows a safe drawing with no contamination hazards.

Another object of the present invention is to make available a device for drawing biological and/or chemical samples, which allows to reduce the risk of misidentifying the sample.

Another object of the present invention is to make available a device for drawing biological and/or chemical samples, which allows easily to perform also external and/or subcutaneous biopsy drawings of biological materials.

A further object of the present invention is to achieve a reduction in the quantity of plastic materials used for the components of the drawing device, as well as in the quantity of transport and/or culture media and/or materials.

These objects and others as well, which shall become more readily apparent from the description that follows, are achieved, in accordance with the present invention, by a device for drawing biological and/or chemical samples, having structural and functional characteristics in accordance with the appended independent claims, additional embodiments thereof being identified in the appended and corresponding dependent claims.

The invention is described in greater detail hereafter with the aid of the drawings, which represent an embodiment provided purely by way of non limiting example.
- Figure 1 shows a partially sectioned lateral view of a first embodiment of the device according to the invention in the position of readiness for sampling.
- Figure 2 is a partially sectioned lateral view of the device of Figure 1 in resting position and closed outwardly.
- Figure 3 is a partially sectioned lateral view of a variant of the device of Figure 1 in resting position, closed outwardly and with the rear part of the rod, which bears the sampling head, removed.
- Figure 4 is a partially sectioned lateral view of the device of Figure 3 in resting position, with the front part, for analysing the drawn sample, removed.
- Figure 5 shows: at its top left, a front view of the bottom of the device of Figure 2; at its top right, a side view of an extractor to be engaged on the bottom of the device to replace the rod in the extraction of the sampling head from the container; at the bottom a front view of the end of the extractor to be engaged on the bottom of the container.
- Figure 6 is a section view of a variant of a device for closing the container according to the invention.
- Figure 7 shows a second embodiment of the device according to the invention in the position of readiness for sampling and with some parts removed.
- Figure 8 shows a first variant of the device of Figure 7, constructed as a scissors-like pincer.
- Figure 9 shows a schematic perspective view of a detail of the device of Figure 7.
- Figures 10 and 11 show additional variants of the device of Figure 1.
- Figure 12 shows two variants of probes usable in the device of Figures 1 through 6.
- Figures 13 (a)-(f) show the steps of usage of the device as per Figures 1 through 6, through to its readying for transport to the laboratory.
- Figures 14 (a)-(c) show the method for extracting the head bearing the sample (to prepare it for laboratory testing) with the aid of the extractor of Figure 5.
- Figures 15 (a)-(c) show a similar method to that of Figure 14, for a device whose rod that bears the sampling head has not been broken.
- Figures 16 (a)-(d) show the method usable to extract the sampling head bearing the sample (to prepare it for laboratory testing) in the case of the device of Figures 3 and 4.
- Figure 17 shows a specific way of using the device in a laboratory analysis.
- Figure 18 shows a use of the closure element of Figure 6.

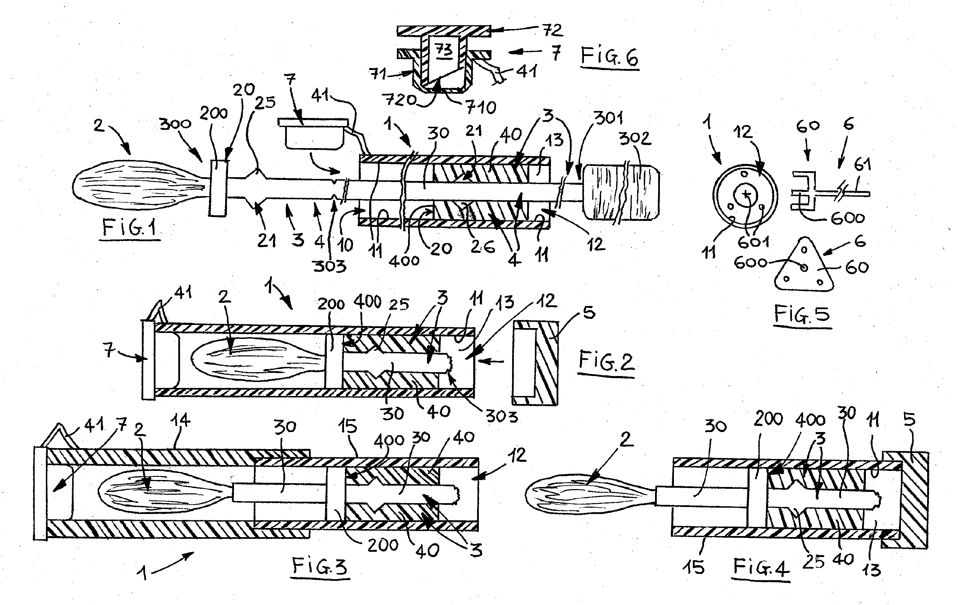

With reference to the figures, the device for drawing biological and/or chemical samples according to the invention, comprising a container 1 and a drawing head 2 that can be at least partially inserted into the container 1 and is movable at least between a position of readiness for drawing, external to the container 1 (e.g., shown in Figures 1, 7, 13(a)-(c)) and a resting position internal to the container 1 (e.g. shown in Figures 2-3, as well as 13(e), 13(f)). Characteristically, it further comprises means 3 for the guided manoeuvring of the head 2 manoeuvring the head 2 in guided fashion relative to the container 1 between the position of readiness for sampling and the resting position.

Control of the position of the head 2 to the container 1 by the guided manoeuvring means 3 assures that there are no fortuitous contacts between the head 2 and the exterior of the container 1, and reduces the risk of fortuitous contacts of the head 2 with the operator or with objects external to the container 1.

Advantageously, the device according to the invention can further comprises connecting means 4 between the head 2 and the container 1, connecting the head 2 to the container 1 for the duration of at least one movement of the head 2 from the position of readiness for sampling to the resting position. The addition of the connecting means 4 and their co-operation, during at least one complete movement of the head 2, with the guided manoeuvring means 3, minimises the risk that, during the sampling operation and the subsequent operations for inserting the head 2 into the container 1, the head 2 itself can come in contact, in fortuitous and unwanted fashion, not only with the outer parts of the container 1, but also with the operator and/or with objects outside the container 1. Moreover, the stable connection between head 2 and container 2 at least until the end of the sampling operation assures that any code affixed on the container 1 exactly corresponds to the drawn sample without any possibility of confusion.

As shall be described hereafter, various embodiments can be provided for the guided manoeuvring means 3, as well as for the connecting means 4, without departing from the scope of the invention. Moreover, advantageously, connecting means 4 can be provided which comprise the guided manoeuvring means 3, the two types of means thereby being mutually integrated to simplify the structure of the device.

In general, within the scope of the invention, various embodiments can be considered. For example, the guided manoeuvring means 3 can be obtained by means of guides and/or sliding sleds, able to be associated more or less removably to the container 1, whereon can slid specifically shaped parts connected to the head 2 (for example, a grip or the like). Said specifically shaped parts can be variously connected to the guides and/or slides, or can be maintained in contact therewith by the action of the operator.

Hereafter shall be described some, preferred embodiments of the device and of its guided manoeuvring means 3 and/or of its connecting means 4 which are, in one aspect or another, particularly advantageous and/or solve specific technical problems linked with sampling.

In general, the container 1 can have various dimensions, according to the type of use. It can be obtained as a common test tube made of plastic material, possibly modified as shall be readily apparent from the description that follows.

In this first part of the description, we shall in particular deal with a first, preferred embodiment of the invention illustrated, in some variants thereof, in Figures 1 through 6, as well as in Figure 12.

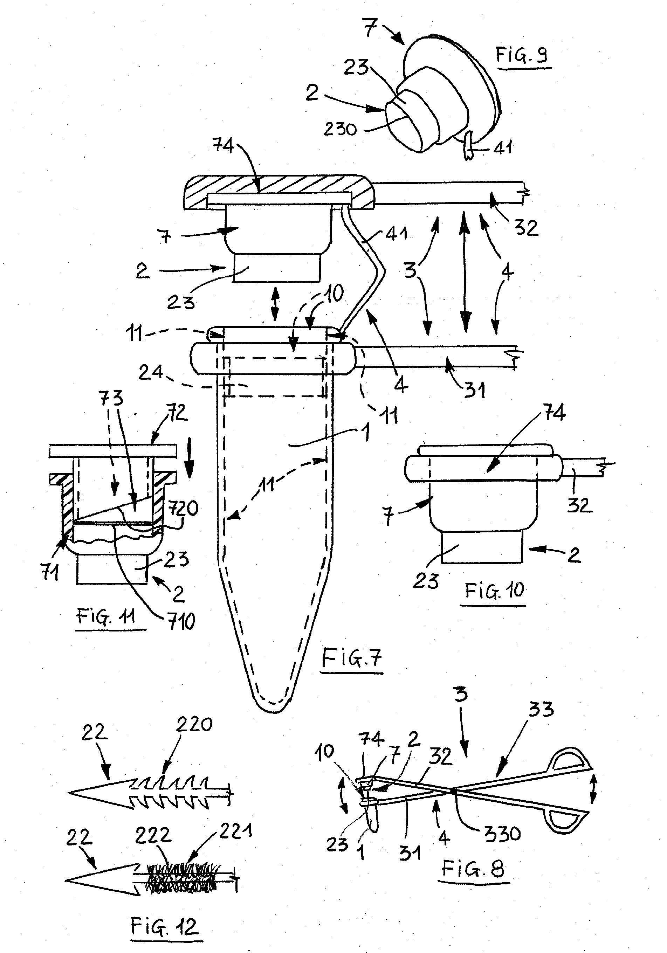

In particular, the device comprises a rod 30, slidable in guided fashion in the container 1 and the bearing the head 2 on its own first end 300. The container 1 comprises an opening 10 for the passage of the head 2, the sliding of the rod 30 bringing the head 2, through the opening 10, from the position of readiness for sampling (shown in Figure 1) to the resting position (illustrated, in particular, also in Figure 2 or 3) and/or vice versa. The rod 30 with its sliding guides, if any, is thus a part of the guided manoeuvring means 3.

Preferably, obviously, the sliding of the rod 30 is sealed (to prevent spills which would contaminate the external environment with the drawn sample, or vice versa, which would contaminate, from the exterior, the environment where the sample will be placed).

The guided manoeuvring means 3 can be further completed by the fact that, as shown in particular in Figure 1, a second end 301 of the rod 30 projects out of the container 1 from the opposite part relative to the opening 10, the rod 30 thereby being manoeuvrable acting on said second end 301, e.g. by means of a grip 302.

The sliding of the rod 30 can be guided directly from lateral walls 11 of the container 1, the head 2 thus having a smaller transverse dimension that that of the rod 30, as in the case of a common syringe provided with a plunger. This solution is not illustrated in the figures. Alternatively, advantageously, to minimise wastage of plastic material, the device further comprises a sliding guide for the rod 30 internal to the container 1 and integral therewith. The sliding guide 40 can be a separate element, fastened to the lateral walls 11 of the container 1 (as shown for example in the figures), or it can be integrated therein by moulding the container 1. The sliding guide 40 (or the lateral walls 11 of the container, if they guide the sliding of the rod 30) can be considered as being a part of the means 3 for the guided manoeuvring of the head 2. Moreover, if the contact between the lateral wall of the rod 30 and the sliding guide 40 (or, alternatively, the lateral walls 11) is sufficiently narrow, the co-operation between the rod 30 and the sliding guide 40 (or the lateral walls 11) can be considered to realise the connecting means 4 between the head 2 and the container 1. In this particular embodiment, therefore, the guided manoeuvring means 3 comprise the connecting means 4.

Advantageously, the sliding guide 40 extends over a predetermined length in the container 1 and is located opposite to the opening 10, in particular in such a way as to leave in the container 1 a space that is sufficient at least to allow, in resting position, the complete housing of the head 2 inside the container 1 (as shown in Figures 2 and 3). In this case, advantageously, the sliding guide 40 can constitute a bottom 12 of the container 1 (if the container 1 does not have its own, wholly independent one).

Preferably, moreover, the device comprises means 20 for the abutment of the head 2, defining the resting position. They can, for example, be obtained on the rod 30, in the form of a widening/narrowing thereof and/or of flange 200, to abut a related counter-flange on the lateral walls 11 of the container, which can also be realised by means of a front wall 400 of the sliding guide 40, as shown in the figures.

Preferably, too, the device comprises means 21 for removably locking the head 2 in its resting position. For example, they can be realised by means of contours 25 and related counter-contours 26 obtained on the rod 30 and on the lateral walls 11 of the container or on the sliding guide 40. Said contours 25 and related counter-contours 26, in particular, can be at least partially deformable elastically to obtain, if the case warrants it, the relative unlocking.

Very advantageously, to close the container 1 after the sampling and when the head 2 has been completely retracted in its interior in the resting position, the device further comprises a sealing closure element 7 able to be inserted into the opening 10. Preferably, the seal provided by the closure element 7 is hermetic.

The closure element 7 can be free or connected to the container 1, for example by means of a connecting tab or brace 41, as in a classic "Eppendorf" test tube.

Advantageously, as shown in Figure 6, the closure element 7 of the container may comprise a lower part 71 and an upper part 72 inserted coaxially in the lower part 71 and defining in combination, together with a membrane 710 of the lower part 71, a chamber 73 for containing a substance that, in particular, can be any substance for the pre-treatment of the biological or chemical sample drawn by means of the head 2 (and which, therefore, is located thereon). For example, the substance can be a desiccant or any transport medium or earth, possibly also liquid or semi-liquid. In the case of samples to be employed for genetic testing (in particular, but not exclusively, for example for genetic traceability tests on control samples, for comparison with reference samples), the substance can be or contain a desiccant which, for example, in turn contains silica and/or, in particular, advantageously, diatom earth. Diatom earth, in addition to its known desiccant properties, useful for the preservation of the sampled biological material, has also been shown to have the ability to bind inhibitors of the PCR ("Polymerase Chain Reaction"), thus aiding the subsequent reactions of extraction and amplification of nucleic acids. Moreover, diatom earth has been shown to have an inhibitory function with respect to the action of some of the factors that in the cellular material exercise an action of breaking the DNA into small pieces.

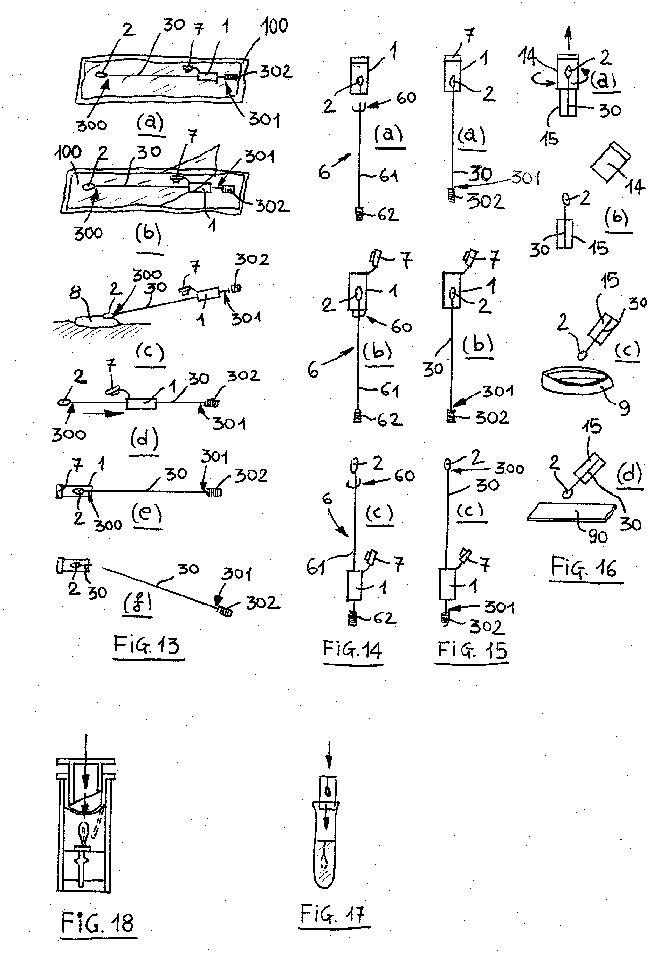

The substance can be maintained in the containment chamber 73, especially if the membrane 710 is semi-permeable or permeable, or it can be released on the head 3 inside the container 1. Advantageously, the upper part 72 is provided with means 720 for perforating or breaking the membrane 710 (for example by means of cutting edges or providing the upper part 72 with a wedge shaped structure oriented towards the membrane 710) for freeing the substance in the container as a result of the insertion by pressure of the upper part 72 into the lower part 71. This system is similar to analogous systems used in the reconstruction of lyophilic drugs immediately before their use and its operation is clearly illustrated in Figure 18 as well.

The head 2 may comprise or be realised (as shown in Figures 1 through 4) by means of a mucous swab. The applicable swab c an be realised with different materials already commonly in use for the fabrication of swabs suitable for different uses, for example: cotton, dacron, nylon, rayon and the like.

The head 2 can comprise or be realised by means of a brush. The brush can be realised with different characteristics and materials, according to the type of sampling.

The mucous swab or the brush can be made of biodegradable material. Said material can comprise or be constituted by calcium alginate. The mucous swab or the brush can also be at least partially made of silica and/or diatom earth.

The head 2 can also comprise, as shown in Figure 12, a needle 22 able to be inserted under the skin and provided with projections 220 able to remove biological material at least during the extraction of the needle 22 from the skin. As shown in Figure 12, the protuberances 220 can be realised by means of the bristles of a brush 221 or of a tube brush 222 associated to the needle 22.

As shown in particular in Figure 13(a), the device thus conceived according to the preferred embodiment described heretofore can also be available in sterile packages 100. It could be inserted therein arranged in an initial position with the head 2 completely extracted from the container 1 and already in position of readiness for sampling.

In this way, once the device is extracted from the package 100, as shown in Figure 13(b), the operator is allowed to perform sampling of a biological and/or chemical matrix 8 as shown in Figure 13(c), gripping the container 1 or the portion of the rod 30 that projects out of the bottom 12 of the container 1 (possibly using the grip 302). Maintaining the same grip, after performing the sampling operation, it will be possible to make the rod 30 slid, as shown in Figure 13(d), until making the head 2 move back to the resting position inside the container 1, as shown in Figure 13(e).

The closure of the container 1 with the sealing closure element 7 (for example a plug) and the characteristics of the bottom 12 of the container 1 (in particular the fact that the sliding guide 40 offers a seal on the rod 30) will allow, providing a hermetic seal, to store the drawn sample in a closed system. The head 2 will be stably maintained in the resting position by the same system that allowed its retraction, possibly assisted by the abutment means 20 and by the removable lock means 21.

Once the head 2 has been retracted in the container 1 in the resting position and the container 1 has been closed with the closure element 7, the rod 30 projects for a good part of its length from the bottom 12 of the container 1, as, for example, shown in Figure 13(e). In these conditions, the sample, contained on the head 2 in the container 1 can be taken to the laboratory (possibly immersed in the pre-treatment substance, if the closure element 7 is structured as in Figures 6 and 18) for tests in the configuration also shown in Figure 15(a). Here the container 1 can be opened, removing the closure element 7, as shown in Figure 15(b), and the head 2 can be extracted from the container 1 acting on the rod 30 (in particular on its second end 301, or grip 302) to the position of readiness for sampling, as shown in Figure 15(c). With the head 2 in this position, maintaining the grip, the operator can swipe the head 2 on a Petri dish 9 or on a microscope slide 90 (as shown schematically respectively in Figure 16(c) and in Figure 16(d), albeit with reference to a variant of this first preferred embodiment of the invention, which we will described immediately hereafter).

Alternatively, since transporting the container 1 with the rod 30 extracted from the bottom 12 can be a wkward, especially when the rod 30 is long, once the device is brought to the condition of Figure 13(e), it can be preferable to break the rod 30 in correspondence with the bottom 12, as shown in Figure 13(f). For this purpose, advantageously, moreover, the rod 30 can comprise means 303 for the facilitate breakage of the rod 30 itself, for example circumferential incisions or the like, located at predetermined distance from the head 2 and which are situated, when the head 2 is in the resting position, outside the container 1 at the opposite part relative to the opening 10 (as, in particular, illustrated in Figure 2, where the rod 30 is already shown broken, with its rear part, comprising the second end 301, removed). Advantageously, the lateral walls 11 of the container 1 can extend for a predetermined length beyond a bottom 12 of the container 1 positioned opposite to the opening 10. This allows to set the container 1 down on a flat surface, in order to assure that any bottom irregularities, due to the breakage of the rod 30, cannot compromise its stability. Moreover, the extension of the lateral walls 11 of the container creates a small chamber 13 which prevents contact with the bottom 12 of the container 1. For a total bio-security guarantee, said small chamber 13 can be limited to the rear by a rubber membrane (not shown) which completely closes the bottom 12 of the container 1 after the breakage of the rod 30. Or, as shown in Figure 2 or in Figure 4, there can be a base element 5 able to be associated to the lateral walls 11 of the container 1 from the side in which they extend beyond the bottom 12 and acting as a closure element on this side of the container 1. The base element 5 can be associated to the lateral walls 1 for example with a mechanical pressure or screw-on coupling and it provides the container 1 with a stable base. Moreover, the presence of the base element 5 provides an additional assurance of seal tightness and the further guarantee that the drawn sample is stored in a closed environment.

At this point, the size of the container 1 is minimised, and the container 1, bearing within it the head 2 (connected to the remaining segment of rod 30) that in turn bears the sample to be tested, can be treated as an ordinary test tube (possibly of the "Eppendorf" type) and easily transported to a laboratory.

Here, after removing the closure element 7, pressing on the segment of rod 30 from the bottom 12 of the container 1 the head 2 can be made to fall directly into the testing environment, as shown in Figure 17 in the case of a testing environment constituted by a test tube.

To facilitate this operation, advantageously, the device comprises an extractor 6 able to be associated to the container 1, for example on the bottom 12 thereof, and able to act on the broken rod 30 to allow the extraction of the head 2 from the container 1. In particular, as shown for example in Figure 5 and in Figures 14(a)-(c), the extractor 6 can comprise an operative end 60, provided with at least a tine 600 (preferably the operative end 60 has more than one) or able to be inserted in an appropriate corresponding seat 601 obtained on the bottom 12 or directly applicable on the segment of rod 30 that projects from the bottom 12. A handle 61, which can be provided with related grip 62 allows easily to handle the extractor 6 which, once it is associated to the bottom 12, replaces the removed part of the rod 30 and allows to extract the head 2 from the container 1, as shown in Figures 15(a)-(c). At this point, possibly handling the system by means of the handle 61 of the extractor 6, one can proceed as if the rod were present and swipe the head 2 on the Petri dish 9 or on the slide 90 or on any other suitable support or container prepared for testing.

In a variant of the device illustrated in Figures 3 and 4, as well as in Figures 16(a)-(d), the container 1 is subdivided in two parts 14, 15 removable from each other in such a way as to uncover the head 2 when the latter is in the resting position. The rod 30 bearing the head 2, in this case remains associated to one of the two parts 14, 15. The two parts 14, 15 of the container 1 can be inserted on each other by pressure, or in screw-on fashion. Alternatively, more economically, the two parts 14, 15 can initially be mutually joined and integrated in the sole container 1, which has a point or area of facilitated breakage at the ideal division between the two parts 14, 15, point or area of facilitated breakage that allows to remove the one of the two parts 14, 15 (designated by the number 14 in the figures) whereto the head 2 with the rod 30 does not remain connected.

In the presence of a container 1 subdivided in the two parts 14, 15, the locking of the motion of the head 2 in the resting position must be achieved by the removable lock means 21 to that the head 2 is at such a distance from the bottom 12 of the container 1 and/or from the sliding guide 40 as to be uncovered when the two parts 14, 15 of the container 1 are separated from each other. This is evidently illustrated in Figures 3 and 4 with reference to a specific embodiment of the removable locking means 21 and of the abutment means 30 of the head 2. The rod 30 in general remains associated to the one of the two parts 14, 15 that comprises the bottom 12 of the container 1 and/or the sliding guide 40 and it is indicated with the number 15 in Figures 3 and 4, as well as 16(a)-(d). In the presence of a container 1 subdivided in two parts 14, 15, it will not even be necessary to use the extractor 6 to extract, for testing purposes, the head 2 from the container 1, but, as shown in Figures 16(a)-(d), it will be sufficient, after removing one of the two parts 14, 15, to grip the one of the parts 14, 15 whereto the head 2 remains connected, to be able to swipe the head 2 on the Petri dish 9 or on the slide 90 or on any other suitable support or container prepared for testing.

The embodiment of the invention illustrated heretofore together with all its variants is particularly suitable for all applications in the fields of cytology, microbiology and medicine, as well as in the forensic field. A sector of particular interest for the application of these devices can also be that of the drawing of biological matrices of animal origin which can be used to perform genetic traceability tests.

In the sector of uterine cytology, where it is necessary to sample matrices that are located inside parts of the body which are not immediately accessible, it is advantageous to use an additional variant of this embodiment of the invention comprises a rigid sheath for the protection of the head 2 (not shown in the figures), coaxial to the rod 30 and movable integrally therewith only until the head 2 is at a predetermined distance from the position of readiness for sampling. Arresting means are also provided (not shown in the figures, but readily comprehensible for those versed in the art) able to block the motion of the rigid sheath in the direction of the position of readiness for sampling beyond said predetermined distance, so that the rod 30 telescopically projects out of the sheath, allowing the head 2 to draw the sample. In this way, the protective sheath covers the head 2 and it is integral with the rod 30 throughout the travel (in both directions) between the resting position and the predetermined distance from the position of readiness to sampling; on the contrary, throughout the travel (in both directions) between the position of readiness for sampling and the predetermined distance therefrom, the protective sheath stops and the rod 30 projects out of the sheath or returns inside the sheath telescopically depending on the direction of motion.

All components of the embodiment of the invention described heretofore and of its variants may be constructed, with appropriate modifications, by means of the same procedures and equipment used for the construction of swabs and test tubes currently on the market. It is believed that large scale production can have extremely limited costs. The device according to this embodiment (and variants thereof) may also be sterilised by means of ethylene oxide or gamma rays, similarly to any other similar medical device and marketed in sterile conditions by means of envelopes. In fact, each component of the device according to this embodiment and its variants doe not have such manufacturing peculiarities as would lead to consider impossible its large scale production. Use of components commonly used for medical and veterinary diagnostics purposes, such as swabs, brushes, as well as sealed structures of known usefulness, such as those similar to the plunger-syringe system, or commonly used equipment such as test tubes, appropriately modified according to the invention, make the device extremely simple and effective.

We shall not describe a second embodiment of the invention, illustrated, together with some of its variants, in Figures 7 through 11. It is particularly effective in the case of sampling of biological matrices of animal origin in the veterinary and animal husbandry fields. In particular, it is specifically suitable for drawing samples from tissue portions, such as parts of the ear or the like, of animals such as cattle, sheep, swine or, in general, farm animals. The biological matrices thus drawn can be used for various purposes, not least of which is the extraction of the DNA for the genetic traceability of the origin of the animal.

With reference in particular to Figures 8 through 11, therefore, in the device according to this embodiment the container 1 comprises an opening 10 and the head 2 comprises a first blade 23 able to be inserted in the container 1. The guided manoeuvring means 3 comprises a first claw 31, whereto is associated the container 1 (for example at an end) and a second claw 32, whereto is associated the first blade 23 (for example at an end). The first and the second claw 31, 32 are movable, relative to one another, from a separated configuration (shown in Figure 7 and in Figure 8), corresponding to the position of readiness for sampling, in which a portion of material to be sampled can be inserted between the first blade 23 and the opening 10, to an approached position (not shown in the figures), corresponding to the resting position, in which the first blade 23 is inserted in the container 1 through opening 10. The movement of the first and of the second claw 31, 32 from the separated position to the approached position takes place through a succession of transitory configurations in which the first blade 23, compressing the portion of material to be sampled in contrast on lateral walls 11 of the container 1 in correspondence with the opening 10 (on the portion of the walls 11 which is at the opening 10), whilst it is inserted into the container 1 cuts a piece f material and causes it to fall through the opening.

Conveniently, the first blade 23 has a closed profile 230, corresponding to the profile of the edge of the cross section of the container at least at the opening 10. In particular, preferably, the profile 230 of the first blade 23 is circular. Optionally and advantageously, the device can also comprise a second blade 24 associated to the lateral walls 11 of the container 1 and operating in contrast on the first blade 23.

Advantageously, the connecting means 4 between the head 2 and the container 1 comprise the first and the second claw 31, 32.

In particular, the first and the second claw 31, 32 can be the claws of a pincer 33. The pincer 33 can be of various types, provided they are suitable for the purpose. In particular, as shown in Figure 8, the pincer 33 is scissors shaped, with its fulcrum 330 peripheral with respect to the container.

Preferably, the container 1 is removable from the first claw 31 and able to be associated thereto by means of appropriate fastening means or by insertion into a corresponding seat (represented in Figure 7 by a ring). In this way, once the container 1 is identified with a code, the head 2 being connected to the container 1 through the relationship between the first and the second claw 31, 32 throughout the movement from the position of readiness for sampling to the resting position, the cut sample that falls into the container 1 is certain to be the one identified by the code on the container 1.

Once the sampling operation is completed, the container 1 can thus be removed from the first claw 31 and closed, for example with a closure element providing a seal (possibly hermetic) able to be inserted into the opening 10.

A new container 1 can be associated to the first claw 31 for a new sampling operation, obviously after replacing or cleaning the first blade 23.

Advantageously and preferably, the closure element 7 is part of the head 2, comprises the first blade 23 and can be removably associated to the second claw 32. In this way, to each container 1 corresponds its own second blade, 23, which remains inserted in the c ontainer 1 at the end of the sampling operation. The sampling action itself, by means of the mutual pressure of the first and of the second claw 31, 32, determines, in addition to the cutting of the sample and to its fall into the container 1, also the closing of the container 1 by the closure element 7.

The closure element 7 can be removably associated to the second branch 32 by appropriate coupling means or by means of a corresponding seat 74 obtained in the second claw 32 (and schematically shown in Figure 7 as a contoured recess and in Figure 10 in the form of a ring into which the closure element 7 is to be inserted). In this way, the closure element can be made integral to the container 1. If the container 1 (as is preferably) is removable from the first claw, then the container 1 (possibly provided with the second blade 24) together with its closure element 7 (possibly provided with the first blade 23) is similar test tube with plug, which can be inserted into the first and into the second claw 31, 32 only at the time of the sampling operation, and be carried away, closed, at the end of the sampling operation with the sample already contained in its interior. In the preferred case in which the closure element incorporates within it the first blade 23 and the head 2, the sampling operation itself closes the container inserting any "contaminated" parts into the container in sealed fashion. If, as is preferable, the device comprise a connecting brace 41 between the closure element 7 and the container 1, the set of the two components can be treated as a common "Eppendorf" test tube, possibly modified by the presence of the first blade 23 or of the first and of the second blade 23, 24.

As in the embodiment described above, and as illustrated in Figure 11, once again the closure element 7 of the container 1 comprises a lower part 71 and an upper part 72 inserted coaxially in the lower part 71 and defining in combination, together with a membrane 710 of the lower part 71, a chamber 73 for containing a substance. Conveniently, the upper part 72 is provided with means 720 for perforating or opening the membrane 710 for releasing the substance into the container as a result of the insertion by pressure of the upper part 72 into the lower part 71. Everything that is stated above in relation both to the substance which can be contained in the containment chamber 73 and to the operation of this particular type of closure element 7 (in particular what is stated previously with reference to Figures 6 and 18) can be applied exactly in this context as well. However, in the previous case, advantageously, if the closure element 7 is part of the head 2 and comprises the first blade 23, the pressure needed to break the membrane 710 can be exercised directly by the compressing action exerted by the first and by the second claw 31, 32 during the cutting, in particular when the closure element 7 is inserted into a seat 74 of the type shown in Figure 7.

The invention achieves important advantages.

The system, thanks to the guided movement of the probe relative to the container, prevents at the root the risk that the operator may come in contact with the collected material and also the risk of indirect and crossed contamination. The fact that the probe and the container are securely connected to each other throughout the time that elapses from the start of the sampling operation to the insertion of the probe into the container, as well as at least for a predetermined time interval preceding the start of the sampling operation, leads to the minimisation of sample identification risks. A greater convenience and ease of execution of the sampling operation is assured, because the operator needs to manoeuvre a single piece. Lastly, there can be a reduction both of the use of plastic material and of the transport and/or culture materials/media of the sample, since the dimensions of the container are not necessarily linked to the dimensions of the probe and of its manoeuvring means.

The invention thus conceived can be subject to numerous modifications and variants, without thereby departing from the scope of the inventive concept that characterises it.

Moreover, all details can be replaced by other technically equivalent elements.

In practice, all materials used, as well as the dimensions, may be any, according to requirements.

## Claims

1. A device for drawing biological and/or chemical samples, comprising a container (1) and a drawing head (2) that can be at least partially inserted into the container (1) and is movable at least between a position of readiness for sampling, external to the container (1) and a resting position internal to the container (1), **characterised in that** it further comprises means (3) for the guided manoeuvring of the head (2), manoeuvring the head (2) in guided fashion relative to the container (1) between the position of readiness for sampling and the resting position.

2. Device as claimed in claim 1, **characterised in that** it further comprises connecting means (4) between the head (2) and the container (1), connecting the head (2) to the container (1) for the duration of at least one movement of the head (2) from the position of readiness for sampling to the resting position.

3. Device as claimed in claim 2, **characterised in that** the connecting means (4) comprise means (3) for guided manoeuvring.

4. Device as claimed in any of the claims 1 through 3, **characterised in that** it comprises a rod (30) able to slide in guided fashion in the container (1) and bearing the head (2) on its own first end (300), the container (1) comprising an opening (10) for the passage of the head (2), the sliding of the rod (30) bring the head (2), through the opening (10), from the position of readiness for sampling to the resting position and/or vice versa.

5. Device as claimed in claim 4, **characterised in that** a second end (301) of the rod (30) projects outside the container (1) at the opposite side with respect to the opening (10).

6. Device as claimed in claim 5, **characterised in that** the rod (30) comprises means (303) for the facilitated breakage of the rod (30) itself positioned at predetermined distance from the head (2) and which are positioned, when the head (2) is in the resting position, outside the container (1) at the opposite part relative to the opening (10).

7. Device as claimed in claim 4, **characterised in that** lateral walls (11) of the container (1) extend for a predetermined length beyond a bottom (12) of the container (1) positioned opposite the opening (10).

8. Device as claimed in claim 7, **characterised in that** it comprises a base element (5) able to be associated to the lateral walls (11) of the container (1) from the side in which they extend beyond the bottom (12) and acting as a measuring element on this side of the container.

9. A device as claimed in any of the claims 6 through 8, **characterised in that** it comprises an extractor (6) able to be associated to the container (1) and able to act on the broken rod (30) to allow the extraction of the head (2) from the container (1).

10. Device as claimed in any of the claims from 4 through 9, **characterised in that** the sliding of the rod (30) occurs in sealed fashion.

11. Device as claimed in any of the claims from 4 through 10, **characterised in that** it further comprises a guide (40) for the sliding of the rod (30) internal to the container (1) and integral therewith.

12. Device as claimed in claim 11, **characterised in that** the sliding guide (40) extends for a predetermined length in the container (1) and is located opposite the opening (10).

13. Device as claimed in any of the claims from 4 through 12, **characterised in that** it comprises abutment means (20) of the head (2), defining the resting position.

14. Device as claimed in any of the claims from 4 through 13, **characterised in that** it comprises means (21) for removably locking the head (2) in the resting position.

15. Device as claimed in any of the claims from 4 through 14, **characterised in that** it comprises an element (7) for sealed closure able to be inserted into the opening (10).

16. Device as claimed in claim 15, **characterised in that** the closure element (7) is connected to the container (1).

17. Device as claimed in any of the claims from 4 through 16, **characterised in that** it comprises: a rigid sheath for protecting the head (2), coaxial to the rod (30) and integrally movable therewith only until the head (2) is at a predetermined distance from the position of readiness for sampling; arresting means able to block the motion of the rigid sheath in the direction of the position of readiness for sampling beyond said predetermined distance, so that the rod (30) telescopically projects out of the sheath, allowing the head (2) to perform the drawing operation.

18. Device as claimed in any of the claims from 4 through 17, **characterised in that** the container (1) is subdivided in two parts (14,15) removable from each other in such a way as to uncover the head (2) when the latter is in resting position, the rod (30) that bears the head (2) remaining associated to one of the two parts (14,15).

19. Device as claimed in claim 18, **characterised in that** the two parts 14, 15 are initially joined to each other and integrated in the single container, which has a point or area of facilitated breakage at the ideal division between the two parts 14, 15, which facilitated breakage point or area allows to remove the one of the two parts 14, 15 whereto the head 2 with the rod 30 does not remain connected.

20. Device as claimed in any of the claims from 4 through 19, **characterised in that** the head (2) comprises a mucous swab.

21. Device as claimed in any of the claims from 4 through 19, **characterised in that** the head (2) comprises a brush.

22. Device as claimed in any of the claims from 4 through 19, **characterised in that** the head (2) comprises a needle (22) able to be inserted under the skin and provided with protuberances (220) able to remove biological material at least during the extraction of the needle (22) from the skin.

23. Device as claimed in claim 22, **characterised in that** the protuberances (220) are obtained by means of a brush (221) or a tube brush (222) associated to the needle (22).

24. Device as claimed in any of the claims 1 through 3, **characterised in that** the container (1) comprises an opening (10), the head (2) comprises a first blade (23) able to be inserted into the container (1), the guided manoeuvring means (3) comprise a first claw (31) whereto is associated the container (1) and a second claw (32) whereto is associated the first blade (23), the first and the second claw (32, 32) being movable, relative to each other, from a separated configuration, corresponding to the position of readiness for sampling, in which a portion of material to be sampled can be inserted between the first blade (23) and the opening (10), to an approached configuration, corresponding to the resting position, in which the first blade (23) is inserted in the container (1) through the opening (10), the movement of the first and of the second claw (31, 32) from the separated position to the approached position occurring through a succession of transitory configurations in which the first blade (23), compressing the portion of material to be sampled in contrast on lateral walls (11) of the container (1) at the opening (10), whilst it is inserted into the container (1) cuts off a piece of material and causes it to fall into the container (1) through the opening (10).

25. Device as claimed in claim 24, **characterised in that** the first blade (23) has a closed profile (230), corresponding to the profile of the edge of the cross section of the container at least in correspondence with the opening (10).

26. Device as claimed in claim 25, **characterised in that** the profile (230) of the first blade (23) is circular.

27. Device as claimed in any of the claims 24 through 26, **characterised in that** it comprises a second blade (24) associated to the lateral walls (11) of the container (1) and operating in contrast on the first blade (23).

28. Device as claimed in any of the claims 24 through 27, **characterised in that** it comprises a sealing closure element (7) able to be inserted into the opening (10).

29. Device as claimed in claim 28, **characterised in that** the closure element (7) is part of the head (2), comprises the first blade (23) and can be removably associated to the second claw (32.

30. Device as claimed in claim 227 or 28, **characterised in that** it comprises a connecting brace (41) between the closure element (7) and the container (1).

31. Device as claimed in claim 15 or 16, or as claimed in any of the claims 24 through 30, **characterised in that** the closure element (7) of the container (1) comprises a lower part (71) and an upper part (72) inserted coaxially in the lower part (71) and defining in combination, together with a membrane (710) of the lower part (71), a chamber (73) for containing a substance.

32. Device as claimed in claim 31, **characterised in that** the upper part (72) is provided with means (720) for perforating or opening the membrane (710) for releasing the substance into the container as a result of the insertion by pressure of the upper part (72) into the lower part (71).

33. Device as claimed in any of the claims 24 through 32, **characterised in that** the connecting means (4) between the head (2) and the container (1) comprise the first and the second claw (31, 32).

34. Device as claimed in claim 33, **characterised in that** the first and the second claw (31,32) are the claws of a pincer (33).

35. Device as claimed in claim 34, **characterised in that** the pincer (33) is scissors-like, with its fulcrum (330) peripheral relative to the container (1).
